# EUROPEAN PATENT APPLICATION

(11) **EP 1 870 395 A1**
(43) Date of publication of application: **26.12.2007**
(21) Application number: 06012531.7
(22) Date of filing: 19.06.2006
(51) Int. Cl.: C07C 217/74, C07C 255/37

(54) **Process for preparation of o-desmethylvenlafaxine and its analogue**

(71) Applicant: KRKA, D.D., Novo Mesto, 8501 Novo mesto (SI)
(72) Inventor: Zupancic, Silvo, 8000 Novo mesto (SI); Skrabanja, Vida, 8000 Novo mesto (SI)
(74) Representative: Becker Kurig Straus

(57) **Abstract**

The present invention belongs to the field of organic chemistry and refers to a process for the preparation of *O*-desmethylvenlafaxine (1-[2-Dimethylamino)-1-(4-hydroxyphenyl)ethyl]cyclohexanol) and its analogues. The invention also relates to a catalytic hydrogenation of cyano-group of the substituted acetonitrile.

## Description

### FIELD OF THE INVENTION

The present invention belongs to the field of organic chemistry and refers to a process for the preparation of *O*-desmethylvenlafaxine (1-[2-Dimethylamino)-1-(4-hydroxyphenyl)ethyl]cyclohexanol) and its analogues. The invention also relates to a catalytic hydrogenation of cyano-group of the substituted acetonitrile.

### BACKGROUND OF THE INVENTION

The antidepressant action of venlafaxine in humans is believed to be associated with its potentiation of neurotransmitter activity in the CNS. Preclinical studies have shown that venlafaxine and its active metabolite, *O*-desmethylvenlafaxine, are potent inhibitors of neuronal serotonin and norepinephrine reuptake and weak inhibitors of dopamine reuptake. Venlafaxine and *O*-desmethylvenlafaxine have no significant affinity for muscarinic, histaminergic, or (alpha)-1 adrenergic receptors in vitro. Pharmacologic activity at these receptors is hypothesized to be associated with the various anticholinergic, sedative, and cardiovascular effects seen with other psychotropic drugs. Venlafaxine and *O*-desmethylvenlafaxine do not possess monoamine oxidase (MAO) inhibitory activity.

The therapy area of venlafaxine is the treatment and prevention of major depressive disorder, anxiety disorder, pain, attention deficit hyperactivity disorder and premenstrual syndrome. Panic disorder, social phobia, cerebral function disorders, obsessive-compulsive disorder, substance abuse, migraines, obesity, weight gain, incontinence are other, but not limited to, potent activities of venlafaxine for treating or preventing above mentioned disorders.

Beside general benefits of venlafaxine several adverse effects among drug-treated patients are enumerated and are referred to affect body as whole, cardiovascular, dermatological, gastrointestinal, nervous, metabolic, respiration and urogenital systems (PDR, Micromedex, Expert Opinion on Investigational Drugs; 6, 1997, 65-78). *O*-desmethylvenlafaxine appears to be equipotent referring its overall action on neurotransmitter uptake and receptor binding but has significant advantages described in the prior art.

Method for preparing *O*-desmethylvenlafaxine as a venlafaxine metabolite is disclosed in EP 112669. Starting precursor was prepared by condensing amide compound in the form of lithium derivative with cyclohexanone in an organic solvent.

The preparation of *O*-desmethylvenlafaxine as described in EP 112669 follows the steps as shown in Scheme I.

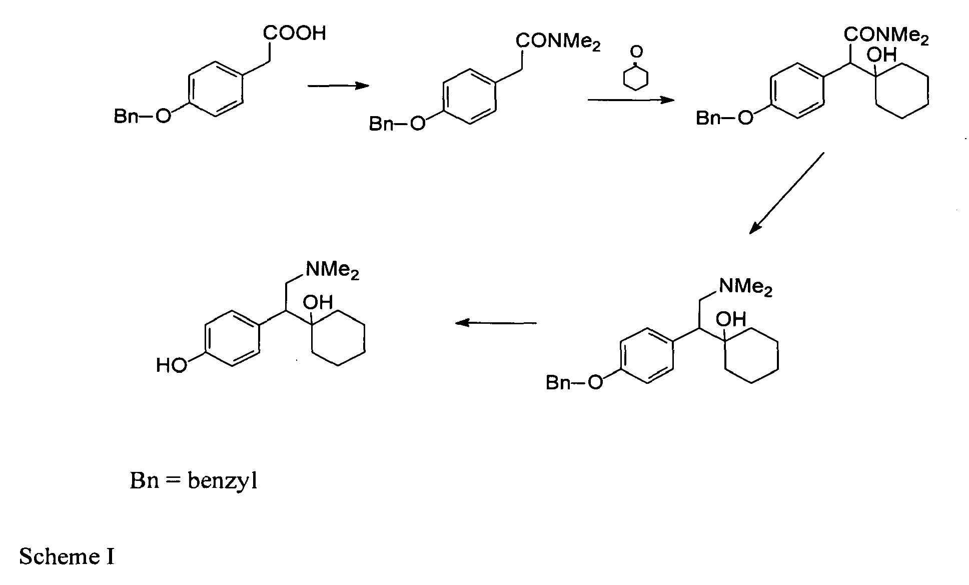

The main drawback of the process is demanding reduction step of amide group.

Another method for the preparation of *O*-desmethylvenlafaxine comprises the step of O-demethylating of venlafaxine and/or its salts following the reduction and *N*-methylation steps (Scheme II).

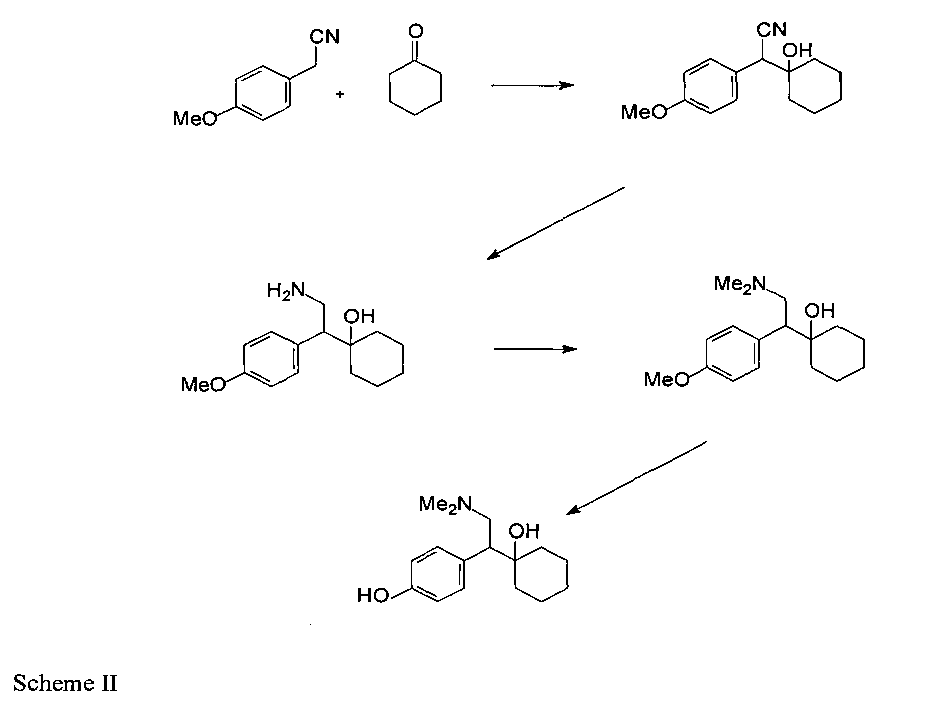

WO 00/59851 disclosed demethylation by contacting venlafaxine with lithium diphenylphosphide to form *O*-desmethylvenlafaxine. Demethylating venlafaxine with a high molecular weight alkane, arylalkyl or arene thiolate anion in a hydroxylic or ethereal solvent is described in WO 03/048104. In general, *O*-demethylation is difficult to carry out routinely.

Reduction of the cyano-group is the key point in the synthesis route for production of venlafaxine and its derivatives. Reduction could be enhanced by different reagents as known from the prior art: EP 112669, EP 1343750, EP 1238967, EP 1249447, WO 03/080560, WO 2004/080934, US 2004/0106818, WO 2006/035457, US 2005/0033088, IP.com Journal 2005, 5 (2) 34, WO 00/59851, CN 1225356.

As presented in WO 03/080560 catalytic hydrogenation of compound 1-[cyano-(p-methoxyphenyl)methyl]cyclohexanol using Pd/C under neutral as well as acidic conditions the reduced retrogression product was obtained.

Most prior art processes for preparing *O*-desmethylvenlafaxine suffer from several drawbacks including demanding hydrogenation and demethylation step. In contrast, the present invention provides a process overcoming these problems by omitting demethylation while preparing *O-*desmethylvenlafaxine. Moreover, reaction conditions are substantially mildly, yields and purity of product are high, and consequently the product is easily recovered. The invention method is economical, safe and scalable to industrial production.

### SUMMARY OF THE INVENTION

As described herein four steps of preparation of *O*-desmethylvenlafaxine have been carried out. In particular, the -OH group of the starting *p*-hydroxybenzylacetonitrile has been protected by the protective groups that are commonly used in organic synthesis (Greene T.W., Wuts P.G.M., Protective groups in organic synthesis, 3rd ed., 1999). Benzyl, substituted benzyl, alkyl, alkenyl, trityl, acetyl, silyl, trialkylsilyl, triarylsilyl, tetrahydropyranyl, tetrahydrofuranyl and carbonates, but not limited to, were particularly selected to temporarily stabilize the -OH group in the p-position of the precursor *p*-hydroxybenzylacetonitrile. 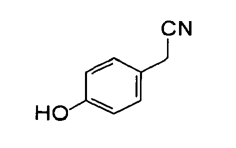 Subsequently, the compound of formula 2 was subjected to condensation with cyclohexanone following the procedure known from the prior art (Sauvetre et al., Tetrahedron, 34, 1978, 2135) and that were commonly used in the preparation of venlafaxine intermediates (EP 112669, Yardley et al., J. Med. Chem., 33, 1990, 2899-2905).

Catalytic hydrogenation by H₂ in the presence of palladium on charcoal and aqueous inorganic acids and organic solvents at ambient temperature has been used for transformation of nitrile group to primary amine.

Conversion of *O*-protected **4** or unprotected *N,N*-didesmethylvenlafaxine 6 to *O*-desmethylvenlafaxine (7) and its analoges may be quickly proceeded by methylation process as known from the prior art (Yardley et al., J. Med. Chem., 33, 1990, 2899-2905; WO 2005/058796).

The protective group has been selectively removed as known from the prior art for example in Greene T.W., Wuts P.G.M., Protective groups in organic synthesis, 3rd ed., 1999. The protecting group may be removed before or after hydrogenation process, as well as contemporarily during catalytic hydrogenation or after methylation.

### DETAILED DESCRIPTION OF THE INVENTION

This invention provides a process for the preparation of pharmaceutically active *O*-desmethylvenlafaxine and its analogues characterised by formula (1): 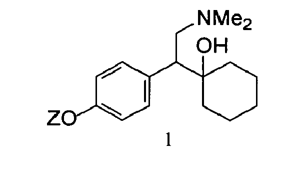 wherein Z is H or any protective group used for the protection of OH group. Protective group used for the protection of OH group is selected from benzyl, substituted benzyl such as p-methoxybenzyl, 3,4-dimethoxybenzyl and o-nitrobenzyl; alkyl such as methyl, ethyl, propyl, butyl, isopropyl; substituated alkyl such as 1-ethoxyethyl, 2,2,2-trichloroethyl, *t*-butyl, allyl; alkenyl; trityl; alkanoyl such as formyl, acetyl, benzylformyl, chloroacetyl, trifluoroacetyl, trichloroacetyl, benzoyl; silyl such as trimethylsilyl, triethylsilyl, triisopropylsilyl, *t*-butyldimethylsilyl, triphenylsilyl; tetrahydropyranyl; tetrahydrofuranyl; methoxymethyl; and carbonates such as methyl carbonate, ethyl carbonate, benzyl carbonate, vinyl carbonate, allyl carbonate, 2,2,2-trichloroethyl carbonate.

That is, the process comprises:
- introducing the protective group on the *p*-hydroxybenzylcyanide 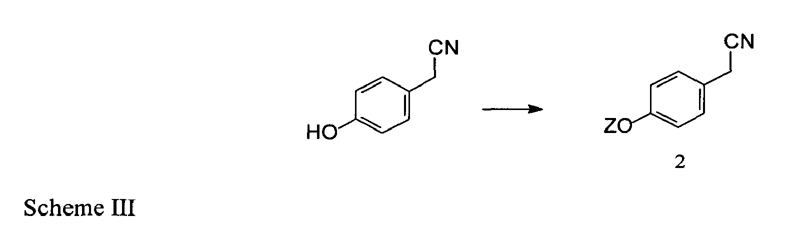
- condensing the protected nitrile 2 with cyclohexanone to give compound 3

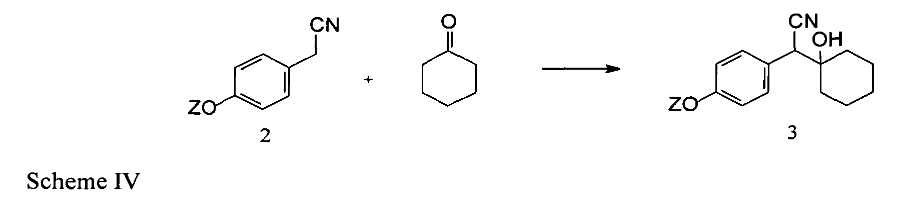

The compound of formula 3 is further
(A) reduced to obtain compound of formula 4 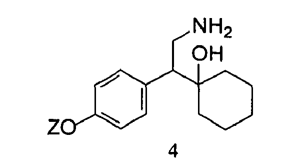 followed by
(B) methylation of amino group and
(C) optionally deprotection of hydroxyl group before step A or before step B or after step B in case of Z ≠ H.
   (C1) In case deprotection takes place before step A the process proceeds according to Scheme V. 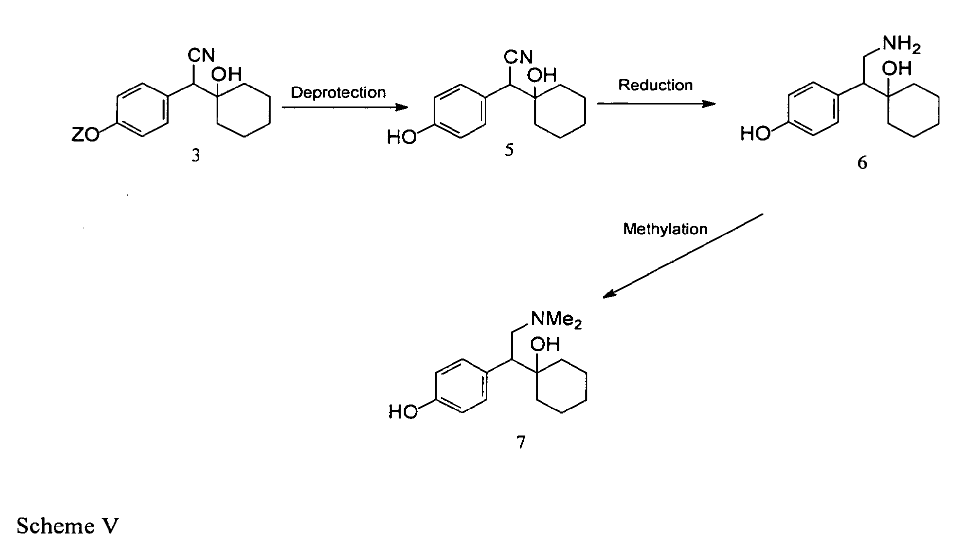
   (C2) In case deprotection takes place after step B the process proceeds according to Scheme VI. 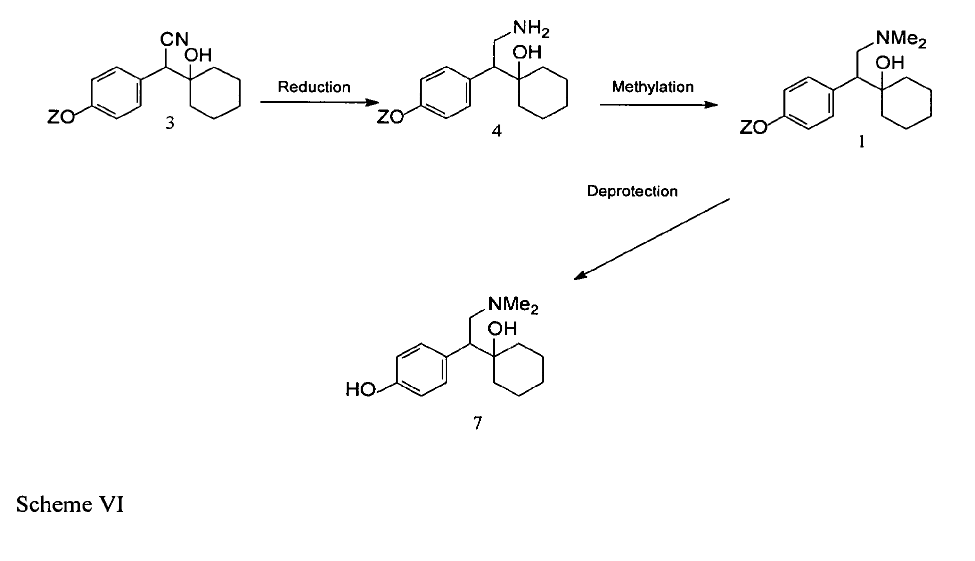
   (C3) In case deprotection takes place before B the process proceeds according to Scheme VII. 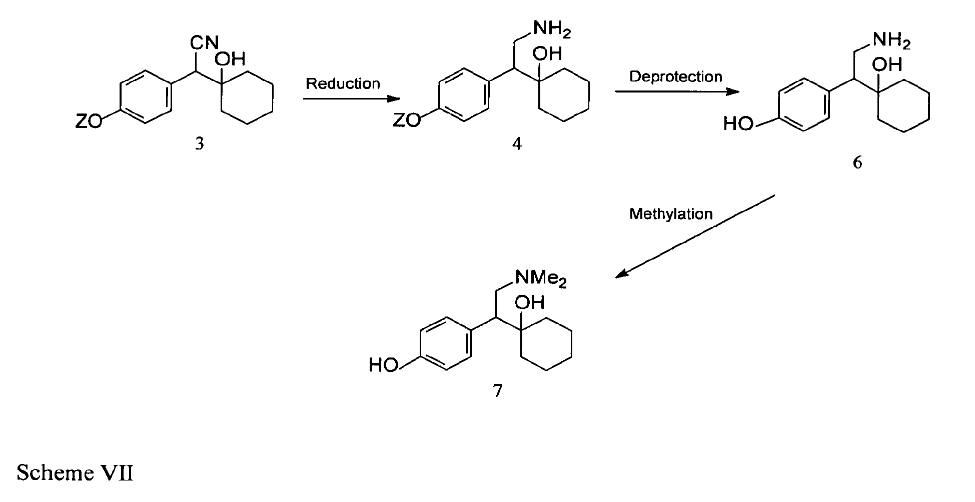
   (C4) In case Z is benzyl, trityl, benzyl carbonate or any protective group which is removed by catalytic hydrogenation) reduction and deprotection are performed in one step (Scheme VIII).

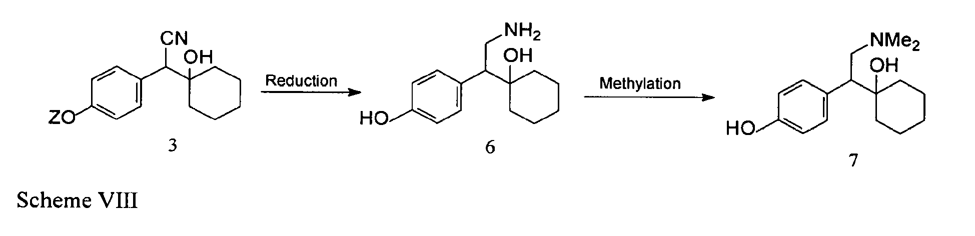

It was surprisingly found that the compound *O*-desmethylvenlafaxine and its analogues characterised by formula (I) can be prepared by a multi step process which comprises the reduction process according to the invention in a very efficient manner and with high yield without formation of substantial amounts of undesired by product or reduced retrogression product. Catalytic hydrogenation by H₂ in the presence of palladium on charcoal, aqueous inorganic acids and organic solvents at ambient temperature has been used for transformation of nitrile group to primary amine. In details, hydrogenation steps in afore described reaction schemes followed the conditions as presented hereinafter.

Cyano-compound **3** is dissolved in an organic solvent selected from alcohols (methanol, ethanol, isopropanol, propanol, butanol and isobutanol), cyclic ethers (THF, dioxane), carbohydrates, DMF, DMA, xylenes and toluene or mixtures of them. Preferably alcohols are used. To the solution the catalyst palladium on charcoal is added. The amount of Pd on charcoal varies from 1% to 20 %, preferably from 5 % to 10 %. The amount of catalyst in the reaction mixture is from 5 % to 50 %, preferably from 10 % to 30 %. Afterwards inorganic acid chosen between HCl, HBr, HI, H₂SO₄, H₃PO₄, HClO₄ or HNO₃ is added, preferably HCl or H₂SO₄. Concentration of the acid is in the range of 20 % to 100%; beneficially aqueous solution of inorganic acid with concentration between 25 % and 50 % was used. At a pressure between 1 and 100 bar, preferably between 2 and 10 bar, and temperature between room temperature and 100 °C, preferably room temperature, the hydrogenation process is let run between 1 h and 10 h, preferably between 2 h and 6 h. After the reaction is completed, the catalyst is removed by filtration. The filtrate is dried. The drying of filtrate could be performed by evaporation of the filtrate and by addition of a second solvent for drying and its evaporation. The solvents used for drying are: isopropanol, isobutanol, toluene, cyclohexane, acetates, and ethers. Afterwards, to the concentrate the organic solvent is added. Preferred are alkanes, alcohols, cycloalkanes, halogenated hydrocarbons, hydrocarbons, ethers, esters, xylenes and toluene. The mixture is cooled and the product as corresponding salt (chloride, bromide, iodide, sulphate, hydrogen sulphate, phosphate, hydrogen phosphate, dihydrogenphosphate, chlorate, nitrate) of compound **4** is separated.

Alternatively, the isolation may be carried out as follows: after the hydrogenation step is completed the reaction mixture is filtered. To the evaporated filtrate water is added and pH is arranged to approximately 10 to 12 by addition of base such as NaOH, KOH, LiOH, NH₃... Then organic solvent selected from the group of alkanes, halogenated alkanes, cycloalkanes, toluene, esters or ethers is added. The phases are separated. The organic phase may be washed and then acidified by addition of appropriate acid to pH approximately 1. This mixture is then dried for example by azeotropic distillation of water. When water is removed from the mixture crystallized product as corresponding salt (chloride, bromide, iodide, sulphate, hydrogen sulphate, phosphate, hydrogen phosphate, dihydrogenphosphate, chlorate, nitrate) of compound 4 is recovered.

The present invention provides an improved process for preparing *O*-desmethylvenlafaxine that differs from prior art. The process of the invention presented herein does not encompass the reduction step of amide as described in EP 112669 (Scheme I) or *O*-demethylation step of venlafaxine as shown Scheme II. The invention comprising the reduction step of nitriles to amines (Schemes V, VI, VII and VIII) by catalytic hydrogenation in the presence of palladium on charcoal, aqueous inorganic acids and organic solvents at ambient temperature results in substantially higher yields in comparison to prior art.

Moreover, reactions conditions are substantially mildly, yields and purity of products are high, and consequently the final product and/or analogues are easily recovered. The invention method is economical, safe and scalable to industrial production.

The process of the present invention is in the following illustrated by examples that should not be construed to limit the scope of the invention in any manner.

### EXAMPLES

### Example 1

0.98 g of (4 mmol) 1-(1-cyano-1-(4-methoxyphenyl)methyl)cyclohexanol was dissolved in 15 ml of methanol. Then 0.6 ml of HCl conc. and 300 mg of 10 % Pd/C were added and hydrogenation at ambient temperature for 6 h at 3.5 bar was performed. After the reaction was completed (HPLC area %: Product 97.32%, Starting compound 1.06%), the mixture was filtered and evaporated to give the title oily residue. Addition of 20 ml isopropanol and evaporation to approximate volume of 2 ml were followed. The mixture was stirred for 3 h at room temperature and 10 h at temperature below 0 °C. The precipitated product was filtered off and yielded 0.95 g of product that corresponded to 83%. HPLC area %: Product 99.7 %.

### Example 2

0.98 g of (4 mmol) 1-(1-cyano-1-(4-methoxyphenyl)methyl)cyclohexanol was dissolved in 15 ml of methanol. Then 2 ml of 2.5 M HCl in ethanol and 300 mg of 10% Pd/C were added and hydrogenation at ambient temperature for 6 h at 3.5 bar was performed. After the reaction was completed (HPLC area %: Product 98.3%, Starting compound 1.0%), the mixture was filtered and evaporated to give the title oily residue. 25 ml of diisopropyl ether was added followed by evaporation to approximately 3 ml. Additionaly, 4ml diisopropyl ether were admixed and stirred for 2 h on ice. After filtration 1.04 g of product (91%) was obtained.
HPLC area %: Product 99.5 %.

### Example 3

0.98 g of (4 mmol) 1-(1-cyano-1-(4-methoxyphenyl)methyl)cyclohexanol was dissolved in 15 ml of methanol. After admixing of 0.8 ml of 37 % H₂SO₄ and 200 mg of 5 % Pd/C the mixture was hydrogenated at ambient temperature for 6 h at 3.5 bar. After the reaction was completed (HPLC area %: Product 97.8%), the mixture was filtered and evaporated. To the resulting oily residue 15 ml of water and 3 M NaOH (pH was adjusted to 12) were added. By adding 15 ml of isopropyl acetate, two phases system was obtained, and after thorough mixing separated. Repeatedly, the aqueous layer was extracted with 10 ml of isopropyl acetate. Combined organic phases were evaporated to 1/₂ volume and acidified to pH below 1. The mixture was then dried by adding and evaporating the isopropyl acetate. Finally, the mixture was evaporated to the approximate volume 3-5 ml and 5 ml of hexane were added. Then stirring for 1 h at room temperature and 1 h in an ice bath were carried out. The precipitated product was filtered off and yielded 1 g of product (88 %).
HPLC area %: Product 98.6%

## Claims

1. A process for the preparation of compound of the formula 1 and salts thereof 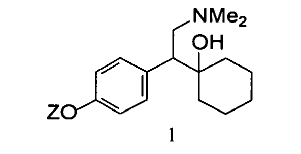 wherein Z is wherein Z is H or any protective group used for the protection of OH group **characterized in that** a compound of the formula 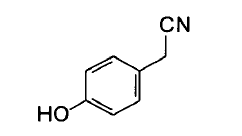 is protected to obtain compound of formula 2 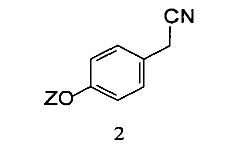 wherein Z is a hydroxy protecting group
followed by condensing the protected compound of formula 2 with cyclohexanone to give compound 3 which is further 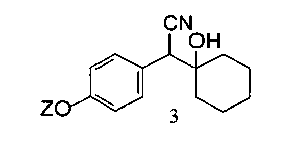
(A) reduced to obtain compound of formula 4 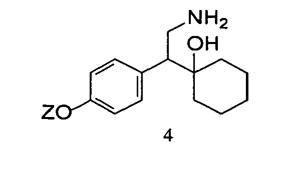 followed by
(B) methylation of amino group and
(C) optionally deprotection of hydroxyl group before step A or before step B or after step B in case of Z # H.

2. A process for the preparation of a compound of the formula 1 and salts thereof 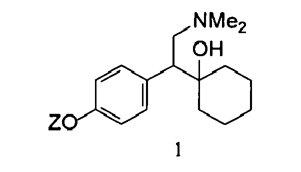 wherein Z is H or any protective group used for the protection of OH group
**characterized in that that** compound of formula 3 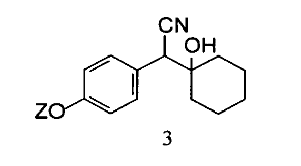 is
(A) reduced to obtain compound of formula 4 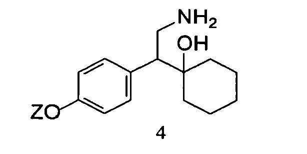 followed by
(B) methylation of amino group and
(C) optionally deprotection of hydroxyl group before step A or before step B or after step B in case of Z ≠ H.

3. The process according to claim 1 or 2,
wherein the protective group used for the protection of the OH group is selected from benzyl, substituted benzyl such as *p*-methoxybenzyl, 3,4-dimethoxybenzyl and *o*-nitrobenzyl; alkyl such as methyl, ethyl, propyl, butyl, isopropyl; substituted alkyl such as 1-ethoxyethyl, 2,2,2-trichloroethyl, *t*-butyl, allyl; alkenyl; trityl; alkanoyl such as formyl, acetyl, benzylformyl, chloroacetyl, trifluoroacetyl, trichloroacetyl, benzoyl; silyl such as trimethylsilyl, triethylsilyl, triisopropylsilyl, *t*-butyldimethylsilyl, triphenylsilyl; tetrahydropyranyl; tetrahydrofuranyl; methoxymethyl; and carbonates such as methyl carbonate, ethyl carbonate, benzyl carbonate, vinyl carbonate, allyl carbonate, 2,2,2-trichloroethyl carbonate.

4. The process according to any of claims 1 to 3 wherein the deprotection step is performed before step A.

5. The process according to any of claims 1 to 3 wherein the deprotection step is performed before step B.

6. The process according to any of claims 1 to 3 wherein the deprotection step is performed after step B.

7. The process according to any of claims 1 to 3 wherein reduction and deprotection are performed in one step.

8. The pProcess according to claim 7 wherein Z is selected from benzyl, trityl, benzyl carbonate or any protective group which is removable by reduction.

9. A process for the preparation of compound with formula 4and its salts 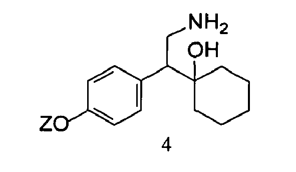 wherein Z is H or any protective group used for the protection of OH group.
**characterized by** that compound of formula 3 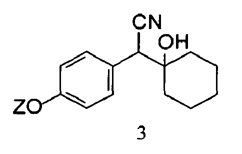 is reduced.

10. The process according to claim 9 wherein protective group Z is selected from H, benzyl, substituted benzyl such as *p*-methoxybenzyl, 3,4-dimethoxybenzyl and o-nitrobenzyl; alkyl such as methyl, ethyl, propyl, butyl, isopropyl; substituated alkyl such as 1-ethoxyethyl, 2,2,2-trichloroethyl, *t*-butyl, allyl; alkenyl; trityl; alkanoyl such as formyl, acetyl, benzylformyl, chloroacetyl, trifluoroacetyl, trichloroacetyl, benzoyl; silyl such as trimethylsilyl, triethylsilyl, triisopropylsilyl, *t*-butyldimethylsilyl, triphenylsilyl; tetrahydropyranyl; tetrahydrofuranyl; methoxymethyl; and carbonates such as methyl carbonate, ethyl carbonate, benzyl carbonate, vinyl carbonate, allyl carbonate, 2,2,2-trichloroethyl carbonate.

11. The process according to claim 9 or claim 10 wherein reduction is catalytic hydrogenation.

12. The process according to claim 11 wherein hydrogenation is catalyzed by palladium on charcoal in the presence of inorganic acid.

13. The process according to any of claims from 9 to 12 wherein hydrogenation is catalyzed by palladium on charcoal in the presence of inorganic acid.

14. The process according to claim 13 wherein the inorganic acid is selected from HCl, HBr, HI, H₂SO₄, H₃PO₄, HClO₄ or HNO₃.

15. The process according to claim 14 wherein inorganic acid is HCl or H₂SO₄.

16. The process according to claim 14 and 15, wherein inorganic acid is used aqueous solution.

17. The process according to any of claims from 9 to 16 wherein the reaction is carried out in an organic solvent selected from alcohols such as methanol, ethanol, isopropanol, propanol, butanol and isobutanol; cyclic ethers such as THF, dioxane; carbohydrates; DMF; DMA; xylenes and toluene or mixtures of them.

18. The process according to claim 17 wherein organic solvent is alcohol.

19. Use of a compound of formula 4 prepared according to process defined by any of claims from 9 to 18 for the preparation of compound of formula 1.
